# EUROPEAN PATENT APPLICATION

(11) **EP 3 246 836 A1**
(43) Date of publication of application: **22.11.2017**
(21) Application number: 17170531.2
(22) Date of filing: 11.05.2017
(51) Int. Cl.: G06F 19/00, G06F 17/20, G06F 17/24, G06Q 50/24, A61B 6/00

(54) **AUTOMATIC GENERATION OF RADIOLOGY REPORTS FROM IMAGES AND AUTOMATIC RULE OUT OF IMAGES WITHOUT FINDINGS**

(30) Priority: 18.05.2016 US 201615158375
(71) Applicant: Siemens Healthcare GmbH, 91052 Erlangen (DE)
(72) Inventor: COMANICIU, Dorin, 08550 Princeton Junction (US); GEORGESCU, Bogdan, 08536 Plainsboro (US); LIU, Wen P., 95125 San Jose (US); ZHOU, Shaohua Kevin, 08536 Plainsboro (US)
(74) Representative: Patentanwälte Bals & Vogel

(57) **Abstract**

A computer-implemented method for automatically generating a radiology report includes a computer receiving an input dataset comprising a plurality of multidimensional patient images and patient information and parsing the input dataset using learned models to determine a clinical domain and relevant image annotations. The computer populates an annotation table using the relevant image annotations and applies one or more domain-specific scriptable rules to populate a report template based on the annotation table. The computer may then generate a natural language radiology report based on the report template.#

## Description

### TECHNOLOGY FIELD

The present invention relates generally to methods, systems, and apparatuses for automatically generating radiology reports from images and automatic rule out of images without findings. Using the disclosed methods, systems, and apparatuses may be applied to the processing for information gathered from a variety of imaging modalities including, without limitation, Computed Tomography (CT), Magnetic Resonance (MR), Positron Emission Tomography (PET), and Ultrasound (US) technologies.

### BACKGROUND

The current standard of practice for reporting in radiology requires clinicians to review each individual slice in a volumetric data set (e.g., MR, CT, or PET) and dictate an oral summary of findings. This dictation is later transcribed into a free-form written report where overall impressions from images are correlated with non-image patient information (i.e. age, patient history). This labor-intensive process is subjective and is not explicit about which image information is used to derive the clinical findings. In addition, many image slices within a volumetric data set do not present findings. Conventional systems do not provide any efficient way to identify and rule out multidimensional (e.g., 2D/3D/4D) images with no findings with high confidence.

### SUMMARY

Embodiments of the present invention address and overcome one or more of the above shortcomings and drawbacks, by providing methods, systems, and apparatuses related to the automatic generation of radiology reports. The main challenges associated with the automatic generation of radiology reports is to manage the complex domain knowledge required to determine the presence of radiologic findings and impressions and to extract the structured and semantic representations of their features from images. To address these challenges, the disclosed techniques apply domain knowledge and learning from existing reports and images in order to determine the necessary image annotations and the associated rules needed to automatically populate clinical report templates. This design transfers the complexity of the semantics in reporting to a set of (over-complete) image annotations.

According to some embodiments, a computer-implemented method for automatically generating a radiology report includes a computer receiving an input dataset comprising a plurality of multidimensional patient images and patient information and parsing the input dataset using learned models to determine a clinical domain and relevant image annotations. The computer populates an annotation table using the relevant image annotations and applies one or more domain-specific scriptable rules to populate a report template based on the annotation table. The computer may then generate a natural language radiology report based on the report template. In some embodiments, the computer receives an indication of a clinical study being performed on the input dataset. The natural language radiology report may then provide an explanation of a clinical finding relevant to the clinical study and one or more image features corresponding to the clinical finding. Once generated, the natural language radiology report may be presented in an interactive graphical user interface which allows a user to retrieve images depicting the one or more image features via activation of one or more links embedded in the natural language radiology report.

The natural language radiology report generated by the aforementioned method may include one or more recommendations for modifying a scanner acquisition protocol to acquire one or more additional patient images. For example, in some embodiments, the computer receives an indication of a clinical study being performed on the plurality of multidimensional patient images and detects that target anatomy relevant to the clinical study is partially or completely out of the field of view of all of the plurality of multidimensional patient images. The aforementioned recommendations may include a recommended modification to patient positioning during imaging.

In some embodiments, during the aforementioned method, the computer applies a rule-out process to the patient images prior to parsing the patient images using the learned models. This rule-out process is performed by receiving an indication of a clinical study being performed on the plurality of multidimensional patient images and identifying a subset of the patient images which are irrelevant to the clinical study. Then, the computer can disregard the subset of the plurality of multidimensional patient images from the input dataset or the input dataset as a whole.

In some embodiments of the aforementioned method, the computer performs an offline preparation process by creating a clinical report template based on existing clinical reports and domain knowledge (e.g., clinical standards, clinical guidelines, or information provided in clinical consults). In some embodiments, the computer uses a basic report template provided in a Radiological Society of North America standardized format to create the clinical report template based on the existing clinical reports and domain knowledge. Once the clinical report template is created, the computer identifies one or more clinical report concepts and acceptable data ranges relevant to the clinical report concepts based on the existing clinical reports and the domain knowledge. During this offline preparation process, the computer also uses the clinical report template, the one or more clinical report concepts, and the acceptable data ranges relevant to the clinical report concepts to create an annotation specification comprising one or more annotation tables and the one or more domain-specific scriptable rules. Additionally, the computer uses the annotation specification to create an annotation system. The computer then applies the annotation system to one or more training images to yield one or more training image annotations and trains one or more image parsing models based on the training images and the training image annotations.

According to other embodiments of the present invention, computer-implemented method for automatically generating a radiology report includes a computer performing an offline training process (similar to that discussed above), along with an online report generation process. The online report generation process includes the computer receiving an input dataset comprising a plurality of multidimensional patient images and patient information and deriving one or more relevant image annotations associated with the input dataset based on the plurality of possible image annotations associated with the clinical domain and the one or more domain specific models. Additionally, during this online report generation process, the computer populates the domain-specific clinical report template using the one or more relevant image annotations and the plurality of scriptable rules and identifies one or more clinically relevant findings based on the populated domain-specific clinical report template. In some embodiments, the computer additionally generates a natural language radiology report based on the clinically relevant findings. This report may then be presented in an interactive graphical user interface. In some embodiments, the computer may also identify and disregard subsets of the patient images which are irrelevant to the clinical domain based on the one or more clinically relevant findings

In some embodiments, during the online report generation process, the computer identifies a change to an existing image acquisition protocol based on the one or more clinically relevant findings. The computer may then communicate with devices to automatically implement the change to the existing image acquisition protocol on an image scanner to acquire one or more new images. Additionally (or alternatively), the change to the existing image acquisition protocol may be displayed in a graphical user interface as a recommendation to a user. The computer may detect that target anatomy relevant to the clinical domain is partially or completely out of the field of view of all of the plurality of multidimensional patient images. The change to the existing image acquisition protocol may alternatively comprise a recommended modification to patient positioning during imaging.

According to other embodiments, a system for automatically generating a radiology report includes a medical information database and one or more databases. The medical information database comprises one or more diagnostic multidimensional (e.g. 2D/3D/4D) image data and non-image patient metadata. The one or more processors are configured to communicate with the medical information database to retrieve a patient-specific input dataset, parse the patient-specific input dataset using learned models to determine a clinical domain and relevant image annotations, and populate an annotation table using the relevant image annotations. The processors are further configured to apply one or more domain-specific scriptable rules to populate a report template based on the annotation table and identify one or more clinically relevant findings based on the populated report template. In some embodiments, the processors may also be configured to generate a natural language radiology report based on the report template. Additionally, the processors may be used to present the natural language radiology report in an interactive graphical user interface which allows a user to retrieve images depicting image features relevant to the clinically relevant findings via activation of one or more links embedded in the natural language radiology report.

Additional features and advantages of the invention will be made apparent from the following detailed description of illustrative embodiments that proceeds with reference to the accompanying drawings.

### BRIEF DESCRIPTION OF THE DRAWINGS

The foregoing and other aspects of the present invention are best understood from the following detailed description when read in connection with the accompanying drawings. For the purpose of illustrating the invention, there is shown in the drawings embodiments that are presently preferred, it being understood, however, that the invention is not limited to the specific instrumentalities disclosed. Included in the drawings are the following Figures:
FIG. 1 provides an overview of a system for automatically generating radiology reports from images, according to some embodiments;
FIG. 2 provides an example graphical user interface (GUI) showing a smart radiology report with embedded links back to image features as described in the findings, as may be generated in some embodiments;
FIG. 3A provides an example of the offline process involved in generating an annotation specification, according to some embodiments;
FIG. 3B presents the information that may be generated during the process illustrated in FIG. 3A for input from the CT cardiac domain;
FIG. 3C presents the information that may be generated during the process illustrated in FIG. 3A for input from the CT abdominal domain;
FIG. 3D provides an example of the offline process involved in generating image processing models, according to some embodiments;
FIG. 3E presents the information that may be generated during the process illustrated in FIG. 3D for input from the CT cardiac and abdominal domains;
FIG. 4A shows a process that may be used to automatically generate radiology reports, according to some embodiments;
FIG. 4B presents a table with examples of input/output data through various steps presented in FIG. 4A;
FIG. 5 illustrates a process for automatically ruling out of images without radiologic findings, according to some embodiments;
FIG. 6 provides an illustration of the processing steps for the online system to generate a sample set of kidney findings, by reasoning and optimal parsing based on multiple data sources including, but not limited to, image features from image analytics, ontologies, prior images & reports, and non-image data.
FIG. 7 illustrates an exemplary computing environment within which embodiments of the invention may be implemented

### DETAILED DESCRIPTION OF ILLUSTRATIVE EMBODIMENTS

The following disclosure describes the present invention according to several embodiments directed at methods, systems, and apparatuses for automatically and efficiently parsing medical image data to derive radiology findings. The disclosed technology can be applied to automatically generate radiology reports by extracting structured report templates and concepts and determine their associated annotation that can be derived from image processing. Additionally, with capabilities to eliminate images without findings, the disclosed technology can be used to adjust scan acquisitions and filter irrelevant images for screening of diseases, such as lung cancer. Furthermore, the use of standardized fields and templates streamlines comparison to longitudinal data and similar cases from the past reports, which allows this system to quickly process and interpret the current patient data in the context of historical big data. The techniques described herein have the potential to not only automate and streamline what is a traditionally a manual task by providing feedback for image acquisitions, eliminating images without findings, but also to elevate the quality of reports by substantiating clinical observations directly with their points of reference in relevant images.

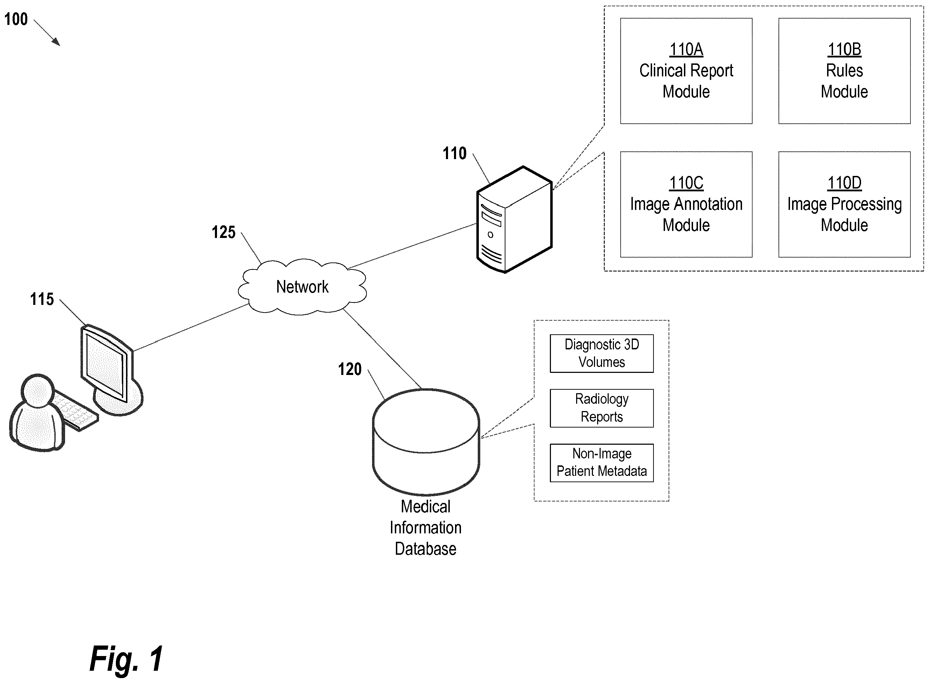

FIG. 1 provides an overview of a system 100 for automatically generating radiology reports from images, according to some embodiments. Briefly, this system 100 applies domain knowledge and learning from existing reports and images in order to determine the necessary image annotations and the associated rules needed to automatically eliminate images without findings and to populate clinical report templates. In this approach, which explicitly defines the valuable correlation between image annotations and clinical interpretations as a set of rules, the system 100 is capable of accommodating different methodologies in image parsing as well as being adaptable to multiple clinical domains.

The system 100 includes a User Computer 115, a Medical Information Database 120, and a Radiology Report Generation Computer 110, all connected via a Network 125. The Network 125 can generally be any computer network or combination of networks generally known in the art. For example, in some embodiments, the User Computer 115 connects over a wired or wireless local area network to the Radiology Report Generation Computer 110. In other embodiments, the Radiology Report Generation Computer 110 may be implemented in a location remote from the location of the User Computer 115. For example, the Radiology Report Generation Computer 110 can be implemented using a "cloud computing" architecture model which allows the User Computer 115 to connect via the Internet.

Medical Information Database 120 comprises diagnostic multidimensional (e.g. 2D/3D/4D) image data, their radiology reports and related non-image patient metadata. The diagnostic multidimensional image data may be captured, for example, using modalities such as Computed Tomography (CT), Magnetic Resonance Imaging (MRI), Positron Emission Tomography (PET) or Ultrasound (US) to support decision making for therapy. These image volumes provide dense anatomical or functional data. Priori requirements include clinical domain knowledge that determines and correlates clinical interpretations (morphological and pathological) with image annotations.

The Radiology Report Generation Computer 110 communicates with the Medical Information Database 120 over the Network 125 to retrieve information to generate an input dataset for generating a radiology report. The exact details of this retrieval may vary, depending on the implementation of the Medical Information Database 120; however, in general any suitable technique generally known in the art may be used. For example, in some embodiments, the information in the Medical Information Database 120 is indexed based on a patient identifier. Thus, by providing this patient identifier to the Medical Information Database 120, all information related to the corresponding patient may be retrieved. It should be noted that the Medical Information Database 120 is only one example of where patient medical information can be stored. In other embodiments, for example, the patient medical information may be stored on the Radiology Report Generation Computer 110 or the information may be provided by the user via the User Computer 115.

The Radiology Report Generation Computer 110 comprises a plurality of modules 110A, 110B, 110C, and 110D which are configured to generate radiology reports from patient data (i.e., both medical image and non-image information) provide image findings that can guide scanner acquisition to improve positioning and protocol. For example, in the case of cone-beam CTs acquired by robotic c-arms, the system 100 may be used to detect when target anatomy is partially or completely out of the field of view and provide improved positioning and protocol in the radiology report. Additionally, in some embodiments the system 100 may be applied to automatically rule out images that do not have any radiologic findings. An offline training process is performed by the modules 110A, 110B, 110C, and 110D to perform tasks such as creating clinical templates, specifications for deriving annotations, and creating image parsing models.

During offline processing, the Clinical Report Module 110A applies domain knowledge and references for each clinical domain to create the basic clinical report template and determine the clinical report concepts (e.g. organ size, organ position, vessel lumen, tissue texture, tissue density, wall thickness, etc.) associated with the template. Additionally, the Clinical Report Module 110A populates each domain report template from all available existing sample reports and determines range of values for all clinical report concepts. Thus, the offline inputs to the Clinical Report Module 110A comprise example reports and domain knowledge (e.g., standards, guidelines, textbook information, information gathered via clinical consults, etc.). The outputs include one or more of a basic clinical template for each domain, a table of clinical report concepts, and possible value ranges (continuous, discrete) for each template. In some embodiments, basic report templates, standardized by the Radiological Society of North America provide the starting point for extracting templates along with various sources of domain knowledge.

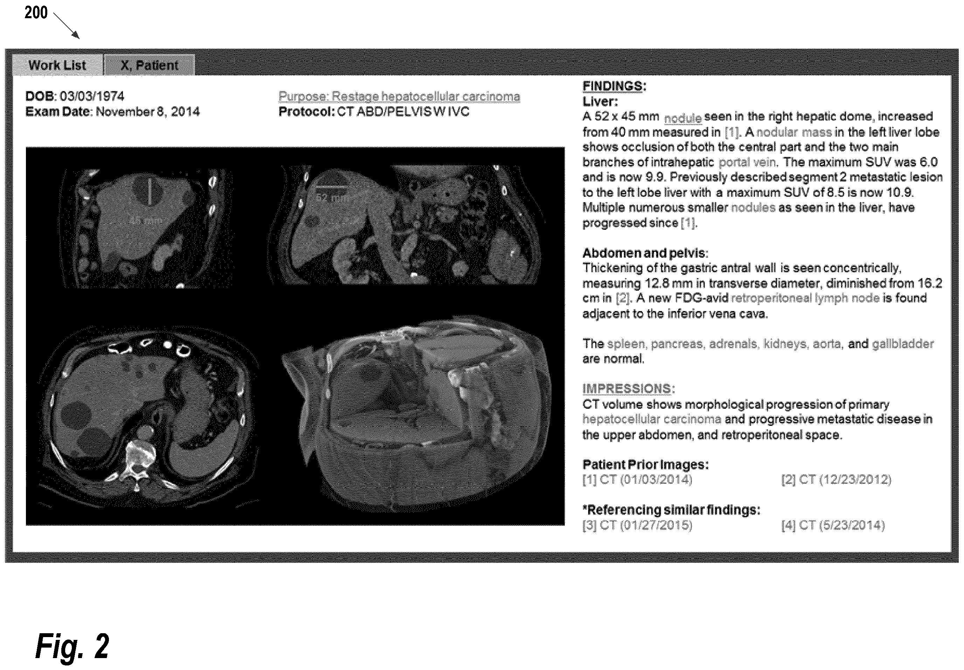

During online processing, the Clinical Report Module 110A uses the populated clinical report template and Natural Language Generation (NLG) to generate the report. As understood in the art, NLG refers to the task of generating natural language from a machine representation system, domain-specific information provided in the template. Thus, the input to the Clinical Report Module 110A during online processing comprises the clinical report template with filled clinical report concept values, while the output is smart report in natural language with embedded links that navigates back to image coordinates of features which correlate with the findings. FIG. 2 provide an example GUI 200 which a smart radiology report on the right with embedded links back to image features as described in the findings.

The Rules Module 110B creates scriptable rules and annotations with clinical report concepts. During offline processing, for each clinical domain and associated basic clinical report template with clinical concepts, the Rules Module 110B determines the image annotations and the corresponding rules which are necessary to generate the values for each of the clinical report concepts. The input provided to the Rules Module for offline processing includes basic report templates, associated clinical report concepts, and their value ranges. The offline output is an annotation specification that may include, for example, scriptable rules and annotation tables. The rules may be implemented using any scripting language generally known in the art (e.g., Python). For example, a generic rule for liver size in natural language could be as follows: Adult male liver with a midclavicular line greater than X cm or a transverse diameter greater than Y cm is considered enlarged. This rule for determining if a liver is enlarged would require the following inputs: clinical information (e.g., age, gender) and an annotated liver volume/mask/mesh from which the system will derive the midclavicular and transverse measurements. The rule would output binary result of 'enlarged' or 'not enlarged'. The output of a rule regarding liver size can be an input to another rule which evaluates the overall normality of the liver. Therefore, a rule can input image derived annotations, non-image information and other rules. A rule can derive additional measurements from given inputs. The output of a rule can be a binary label, a classification, a range in measurements, etc.

During online processing, the Rules Module 110B select the clinical domain report template and apply corresponding rules to fill the template with clinical reports concept values (online). Thus, given the annotation values, the Rules Module 110B uses the scriptable rules generated offline to select the clinical report template and generate the values for associated clinical report concepts. The online inputs to the Rules Module 110B comprise the filled annotation table, scriptable rules, report templates, and clinical report concepts. The output is the clinical report template filled with concept values.

Given the annotation specification, the Image Annotation Module 110C builds the image annotation system and uses it to annotate sample images. It should be noted that the Image Annotation Module 110C only operates offline. The input to the Image Annotation Module 110C includes the annotation specification (i.e., scriptable rules and annotation tables), images, and patient information, while the output is the annotation system and image annotations.

The Image Processing Module 110D training image parsing models and uses these models to determine domain, modality and annotations. During offline processing, the Image Processing Module 110D determines what is the optimal algorithm or optimal way to sequentially determine the annotation values (scalable to large number of annotations, and includes for example: determining the domain, image type, etc.). Here, the input is the annotated images/non-image patient information and output is one or more optimal (hierarchical) image parsing models. Examples of image parsing models that may be generated by the Image Processing Module 110D include, without limitation, discriminative classifiers (probabilistic boosting trees (PBT), marginal space learning (MSL), marginal space deep learning (MSDL), neural networks (NN), etc.), regression models, hierarchical models, statistical shape models, probabilistic graphical models, etc. Examples of methods that learn and represent the hierarchical structure of complex domains, include reinforcement learning, recurrent neural networks (RNN), deep Q-learning, statistical modeling, etc.

During online processing, the Image Processing Module 110D scans the input image to determine the annotation values using the trained image parsing models. The annotation values output by the Image Processing Module 110D may be represented, for example, in a completed annotation table (including domain, image type, etc.).

Various interfaces may be used to facilitate the communications between the User Computer 115 and the Radiology Report Generation Computer 110. For example, in some embodiments, the Radiology Report Generation Computer 110 supports a web application which displays a graphical user interface (GUI) in a webpage on the User Computer 115. The User can then interact with the Radiology Report Generation Computer 110. In other embodiments, the Radiology Report Generation Computer 110 may be configured to accept commands via a custom application programming interface (API). Thus, for example, a development tool installed on the User Computer 115 may be configured to use the API in generating and displaying radiology reports.

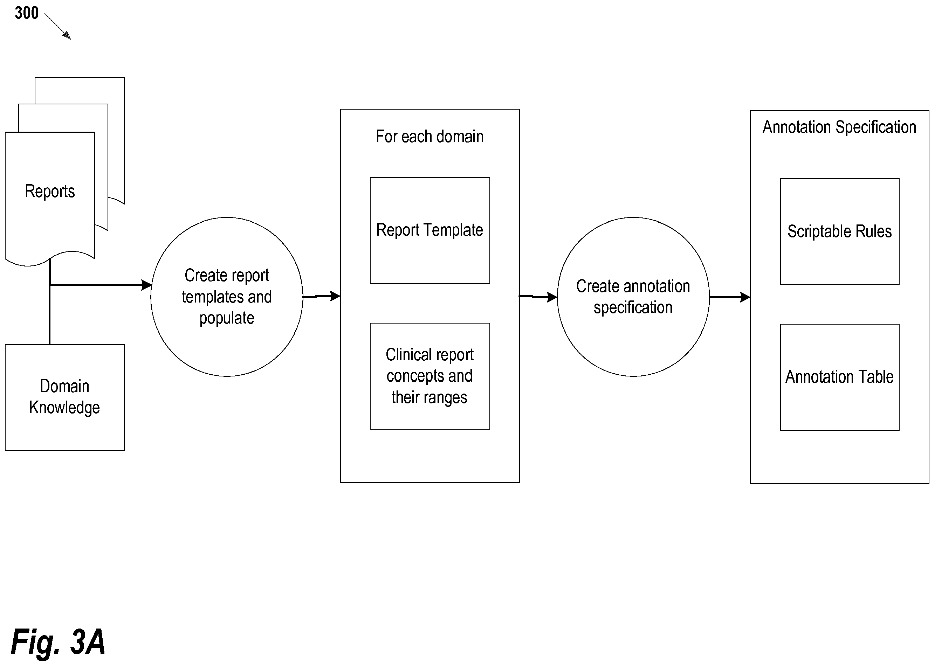

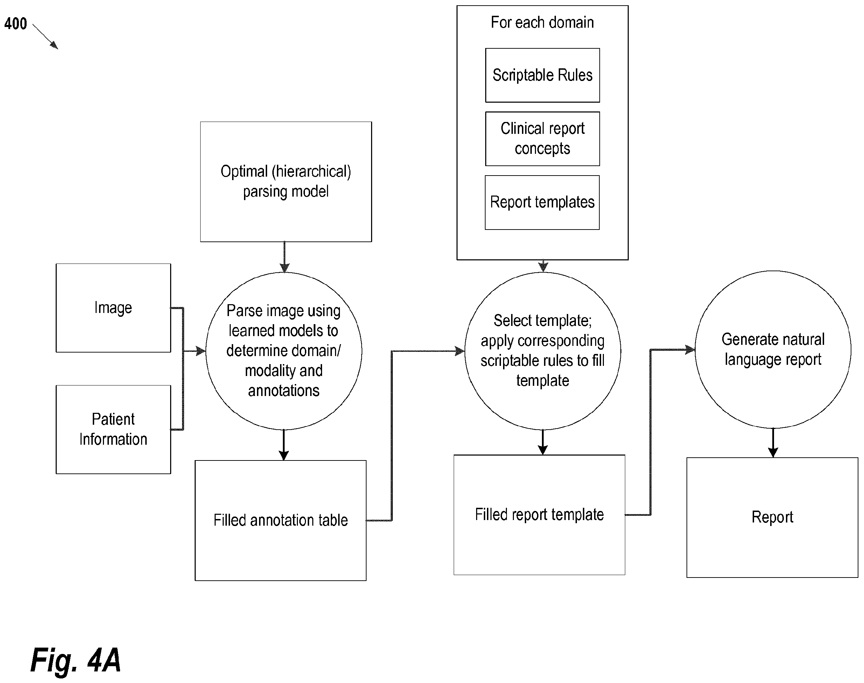

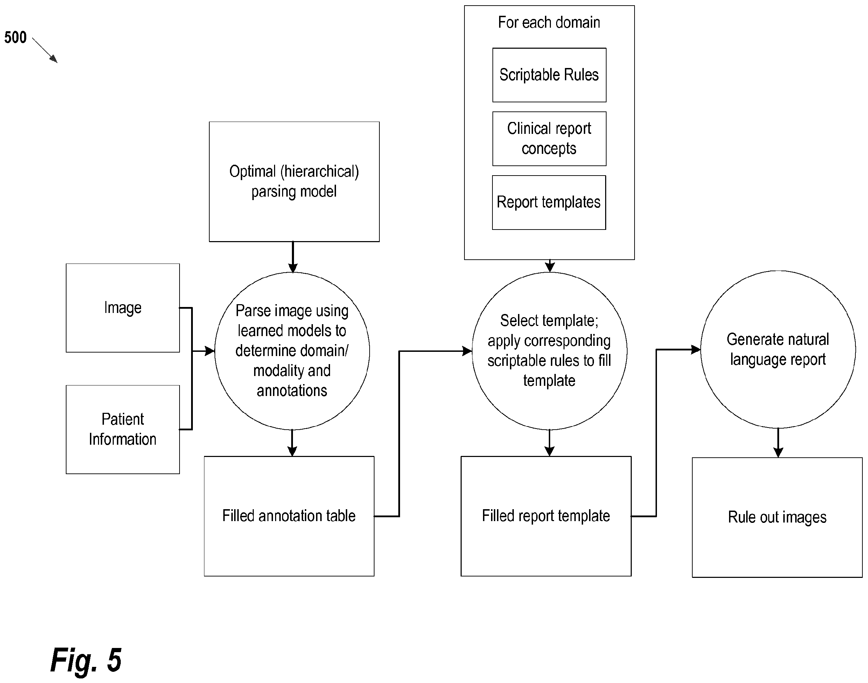

The system 100 illustrated in FIG. 1 overcomes many problems associated with the conventional medical information tracking systems by automatically eliminating images without findings and generation of radiology reports through a flexible, scalable system with structure and transparency of the relationship of images and clinical interpretations. To clarify steps performed by each module presented in FIG. 1, the figures set out below provide examples in two sample clinical domains for CT Cardiac and CT Abdominal (see FIGS. 3A - 3E and FIGS. 4A - 4C), however the design of the system can be easily extended for additional radiology domains. For ruling-out images without radiologic findings, the same architecture applies. However, instead of creating a clinical report, a particular slice can be flagged if it is determined to provide no features linked to any findings that would be included in a resultant report (FIG. 5).

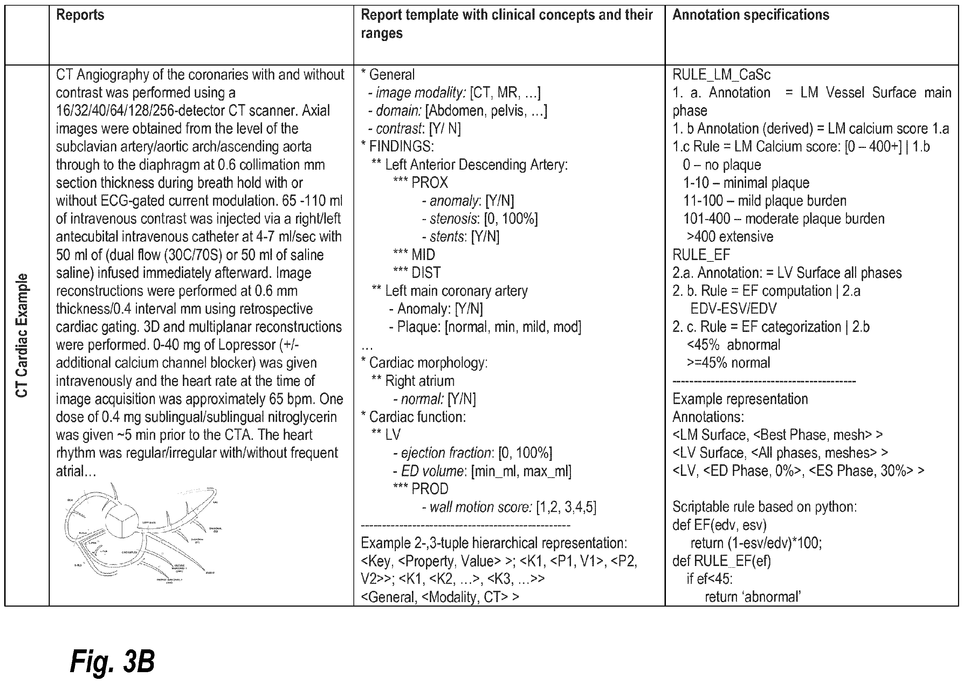

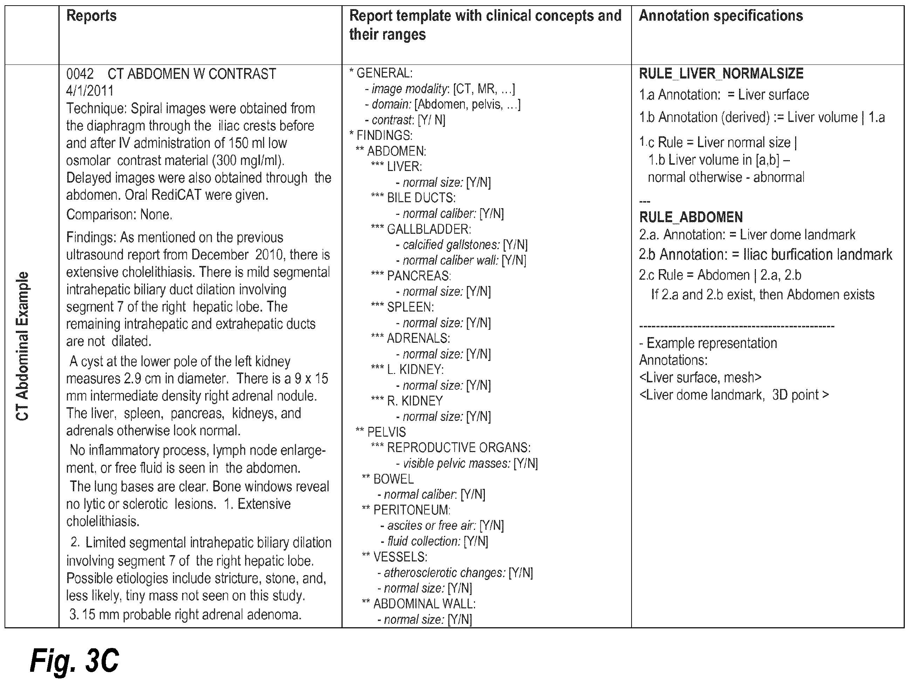

FIG. 3A provides an example of the offline process 300 involved in generating an annotation specification, according to some embodiments. Specifically, report templates are created and populated based on reports and domain knowledge. Then, using these report templates with clinical concepts and their ranges, an annotation specification is created. FIGS. 3B and 3C present the information that may be generated during the process illustrated in FIG. 3A. The information presented in FIG. 3B corresponds to processing of a CT cardiac input dataset, while FIG. 3C shows the results for a CT abdominal input dataset.

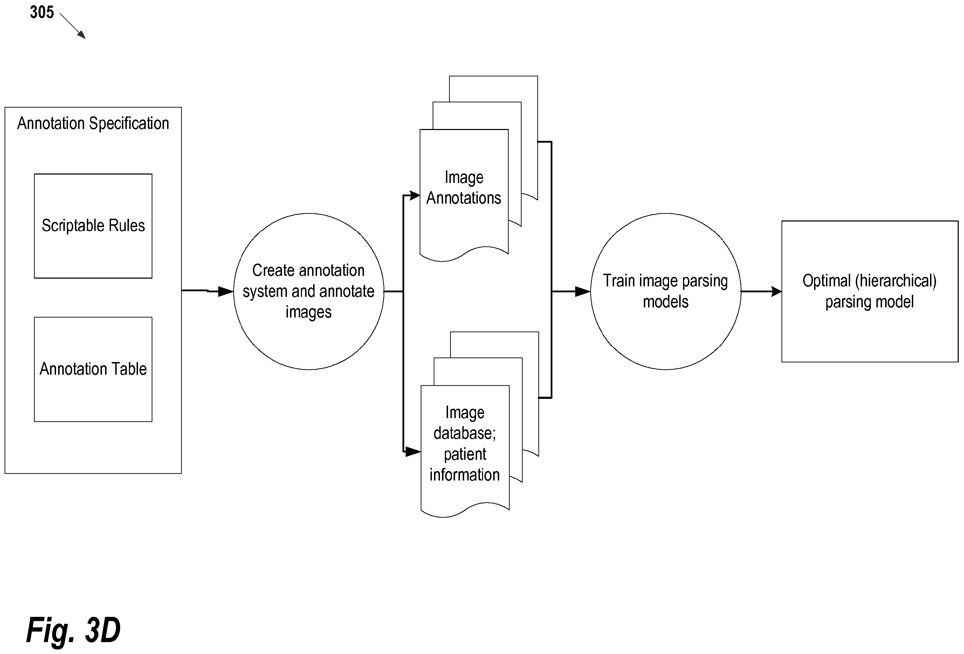

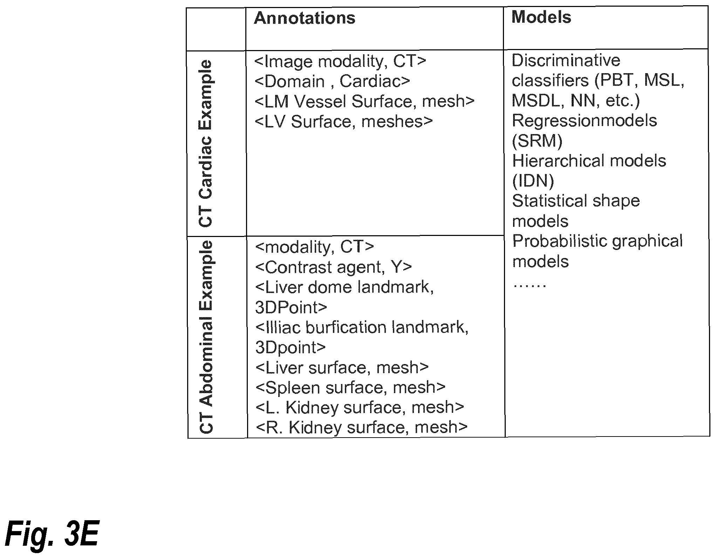

FIG. 3D provides an example of offline process 305 involved in generating image processing models, according to some embodiments. Again, the functionality described above in FIG. 1 with reference to the Radiology Report Generation Computer 110. First, an annotation system is created and used to annotated images. Then image annotations are then used to train image paring models. FIG. 3E presents a table showing the input/output data through various steps of the process shown in FIG. 3D, for CT cardiac and CT abdominal input data sets.

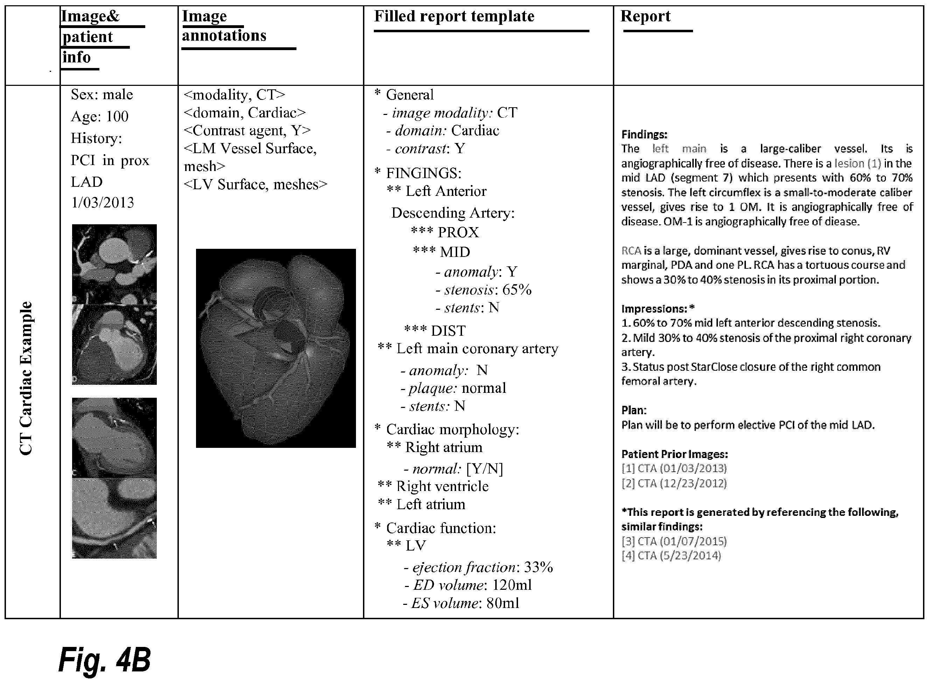

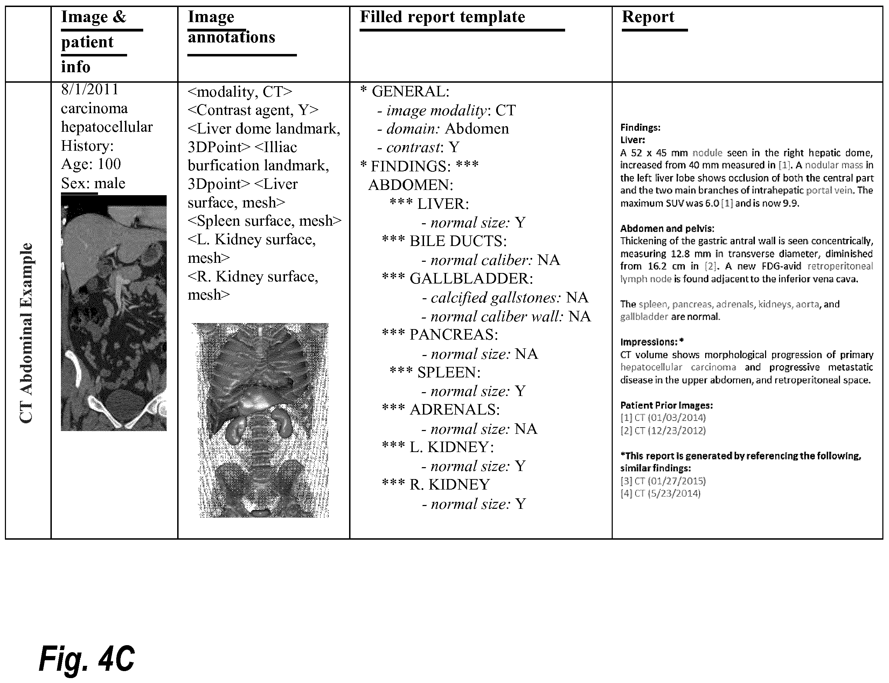

FIGS. 4A and 4B provide an example of online processing according to some embodiments of the present invention. FIG. 4A shows a process 400 applied by the online system architecture and their associated input/output requirements, according to some embodiments. Briefly, an image is parsed using learned models to determine domain/modality information, as well as related annotates. Next, a template is selected and filled using corresponding scriptable rules. Then, a natural language report is generated based on the filled report template. Using CT cardiac as a sample radiology report domain, FIG. 4B presents a table with examples of input/output data through various steps presented in FIG.4A. FIG. 4C provides a table showing the data associated with applying the process shown in FIG. 4A to the CT abdominal domain.

FIG. 5 illustrates a process 500 applied by the online system architecture and the associated input/output requirements for automatically ruling out of images without radiologic findings, according to some embodiments. Many image slices within a volumetric data set do not present findings. Therefore, for processes that require a review of each individual slice, a system that can identify and rule out slices with no findings with high confidence has the potential to impact workflow efficiency. For example, these features can be used to filter out irrelevant images during cancer screenings. By automatically identifying images without findings, such a system can help radiologists focus on pertinent images. Furthermore, images acquired for suspicions of one disease can also be automatically processed to rule-out the presence of other diseases, in order to provide more value through comprehensive screening. In the example of FIG. 5, images are first parsed with learned models to determine domain/modality annotations. The annotations are then used to select a template and fill it with corresponding scriptable rules. Using the filled report template as a guide, images without radiologic findings are flagged.

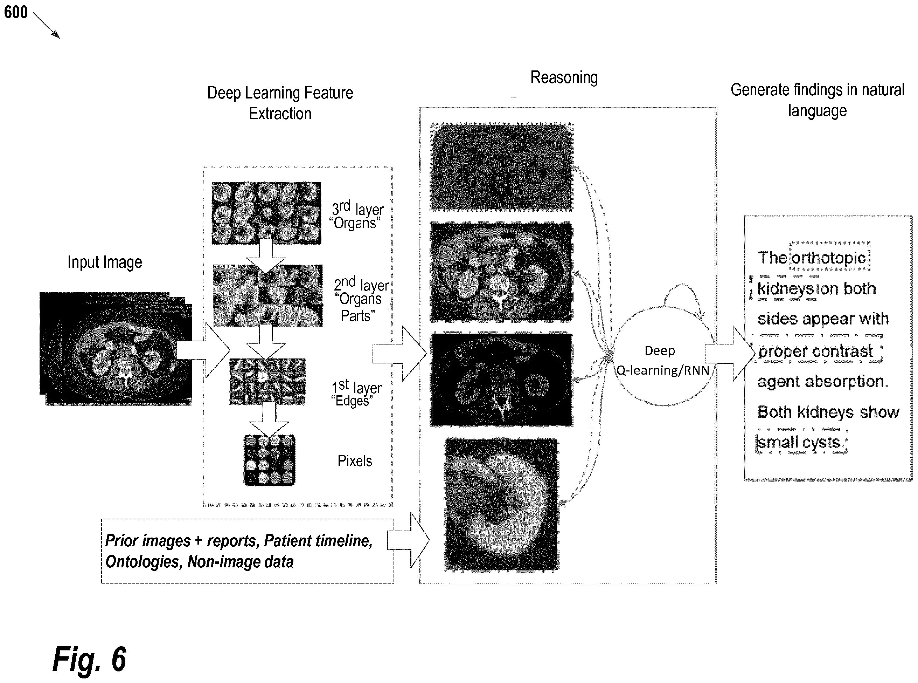

FIG. 6 illustrates the processing steps 600 associated with generating a sample set of kidney findings, according to some embodiments. Briefly, this example is divided into three stages: deep learning and feature extraction, reasoning, and generation of a natural language representation of the findings. These stages correspond to functionality provided by the Image Processing Module 110D, the Rules Module 110B, and the Clinical Report Module 119A, respectively. In FIG.6, input images of a patient's kidneys are received. Deep learning is then applied to the images on a plurality of layers (down to the pixel level) in order to extract features. These features are then used, along with other relevant patient information in a reasoning algorithm. The reasoning algorithm then outputs a natural language report describing the image data. Note that the report includes certain words or phrases that are highlighted by boxes. Such highlighting maybe used to draw a clinician's attention to important information and allow the report to be quickly parsed. Additionally, in some embodiments these words are phrased to important information in the input dataset. For example, the phrase "small cysts" is highlighted in the report shown in FIG. 6. In this case, a user clicking on the phrase may cause the computer displaying the report to retrieve and display one or more images that show the cysts. In this way, it should be understood that the output report is interactive and allows the user to review not only the conclusions presented in the report, but also the basis for those conclusions.

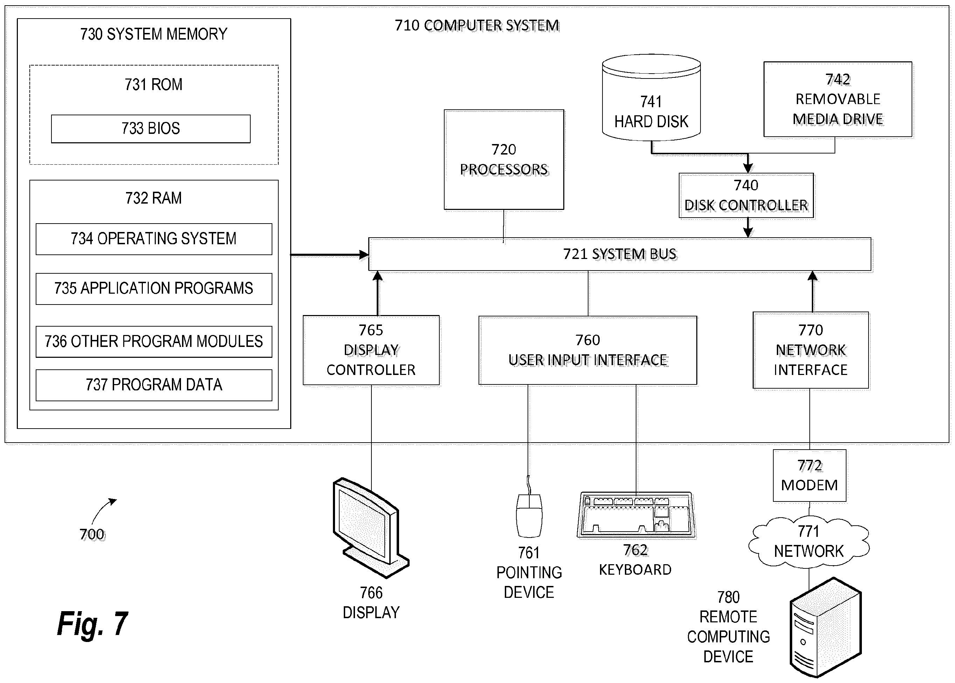

FIG. 7 illustrates an exemplary computing environment 700 within which embodiments of the invention may be implemented. For example, computing environment 700 may be used to implement one or more components of system 100 shown in FIG. 1. Computers and computing environments, such as computer system 710 and computing environment 700, are known to those of skill in the art and thus are described briefly here.

As shown in FIG. 7, the computer system 710 may include a communication mechanism such as a system bus 721 or other communication mechanism for communicating information within the computer system 710. The computer system 710 further includes one or more processors 720 coupled with the system bus 721 for processing the information.

The processors 720 may include one or more central processing units (CPUs), graphical processing units (GPUs), or any other processor known in the art. More generally, a processor as used herein is a device for executing machine-readable instructions stored on a computer readable medium, for performing tasks and may comprise any one or combination of, hardware and firmware. A processor may also comprise memory storing machine-readable instructions executable for performing tasks. A processor acts upon information by manipulating, analyzing, modifying, converting or transmitting information for use by an executable procedure or an information device, and/or by routing the information to an output device. A processor may use or comprise the capabilities of a computer, controller or microprocessor, for example, and be conditioned using executable instructions to perform special purpose functions not performed by a general purpose computer. A processor may be coupled (electrically and/or as comprising executable components) with any other processor enabling interaction and/or communication there-between. A user interface processor or generator is a known element comprising electronic circuitry or software or a combination of both for generating display images or portions thereof. A user interface comprises one or more display images enabling user interaction with a processor or other device.

Continuing with reference to FIG. 7, the computer system 710 also includes a system memory 730 coupled to the system bus 721 for storing information and instructions to be executed by processors 720. The system memory 730 may include computer readable storage media in the form of volatile and/or nonvolatile memory, such as read only memory (ROM) 731 and/or random access memory (RAM) 732. The RAM 732 may include other dynamic storage device(s) (e.g., dynamic RAM, static RAM, and synchronous DRAM). The ROM 731 may include other static storage device(s) (e.g., programmable ROM, erasable PROM, and electrically erasable PROM). In addition, the system memory 730 may be used for storing temporary variables or other intermediate information during the execution of instructions by the processors 720. A basic input/output system 733 (BIOS) containing the basic routines that help to transfer information between elements within computer system 710, such as during start-up, may be stored in the ROM 731. RAM 732 may contain data and/or program modules that are immediately accessible to and/or presently being operated on by the processors 720. System memory 730 may additionally include, for example, operating system 734, application programs 735, other program modules 736 and program data 737.

The computer system 710 also includes a disk controller 740 coupled to the system bus 721 to control one or more storage devices for storing information and instructions, such as a magnetic hard disk 741 and a removable media drive 742 (e.g., floppy disk drive, compact disc drive, tape drive, and/or solid state drive). Storage devices may be added to the computer system 710 using an appropriate device interface (e.g., a small computer system interface (SCSI), integrated device electronics (IDE), Universal Serial Bus (USB), or FireWire).

The computer system 710 may also include a display controller 765 coupled to the system bus 721 to control a display or monitor 766, such as a cathode ray tube (CRT) or liquid crystal display (LCD), for displaying information to a computer user. The computer system includes an input interface 760 and one or more input devices, such as a keyboard 762 and a pointing device 761, for interacting with a computer user and providing information to the processors 720. The pointing device 761, for example, may be a mouse, a light pen, a trackball, or a pointing stick for communicating direction information and command selections to the processors 720 and for controlling cursor movement on the display 766. The display 766 may provide a touch screen interface which allows input to supplement or replace the communication of direction information and command selections by the pointing device 761.

The computer system 710 may perform a portion or all of the processing steps of embodiments of the invention in response to the processors 720 executing one or more sequences of one or more instructions contained in a memory, such as the system memory 730. Such instructions may be read into the system memory 730 from another computer readable medium, such as a magnetic hard disk 741 or a removable media drive 742. The magnetic hard disk 741 may contain one or more data stores and data files used by embodiments of the present invention. Data store contents and data files may be encrypted to improve security. The processors 720 may also be employed in a multi-processing arrangement to execute the one or more sequences of instructions contained in system memory 730. In alternative embodiments, hard-wired circuitry may be used in place of or in combination with software instructions. Thus, embodiments are not limited to any specific combination of hardware circuitry and software.

As stated above, the computer system 710 may include at least one computer readable medium or memory for holding instructions programmed according to embodiments of the invention and for containing data structures, tables, records, or other data described herein. The term "computer readable medium" as used herein refers to any medium that participates in providing instructions to the processors 720 for execution. A computer readable medium may take many forms including, but not limited to, non-transitory, non-volatile media, volatile media, and transmission media. Non-limiting examples of non-volatile media include optical disks, solid state drives, magnetic disks, and magneto-optical disks, such as magnetic hard disk 741 or removable media drive 742. Non-limiting examples of volatile media include dynamic memory, such as system memory 730. Non-limiting examples of transmission media include coaxial cables, copper wire, and fiber optics, including the wires that make up the system bus 721. Transmission media may also take the form of acoustic or light waves, such as those generated during radio wave and infrared data communications.

The computing environment 700 may further include the computer system 710 operating in a networked environment using logical connections to one or more remote computers, such as remote computing device 780. Remote computing device 780 may be a personal computer (laptop or desktop), a mobile device, a server, a router, a network PC, a peer device or other common network node, and typically includes many or all of the elements described above relative to computer system 710. When used in a networking environment, computer system 710 may include modem 772 for establishing communications over a network 771, such as the Internet. Modem 772 may be connected to system bus 721 via user network interface 770, or via another appropriate mechanism.

Network 771 may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 710 and other computers (e.g., remote computing device 780). The network 771 may be wired, wireless or a combination thereof. Wired connections may be implemented using Ethernet, Universal Serial Bus (USB), RJ-6, or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 771.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

A graphical user interface (GUI), as used herein, comprises one or more display images, generated by a display processor and enabling user interaction with a processor or other device and associated data acquisition and processing functions. The GUI also includes an executable procedure or executable application. The executable procedure or executable application conditions the display processor to generate signals representing the GUI display images. These signals are supplied to a display device which displays the image for viewing by the user. The processor, under control of an executable procedure or executable application, manipulates the GUI display images in response to signals received from the input devices. In this way, the user may interact with the display image using the input devices, enabling user interaction with the processor or other device.

The functions and process steps herein may be performed automatically or wholly or partially in response to user command. An activity (including a step) performed automatically is performed in response to one or more executable instructions or device operation without user direct initiation of the activity.

The system and processes of the figures are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. As described herein, the various systems, subsystems, agents, managers and processes can be implemented using hardware components, software components, and/or combinations thereof. No claim element herein is to be construed under the provisions of 35 U.S.C. 112, sixth paragraph, unless the element is expressly recited using the phrase "means for."

## Claims

1. A computer-implemented method for automatically generating a radiology report, the method comprising:
receiving, by a computer, an input dataset comprising a plurality of multidimensional patient images and patient information;
parsing, by the computer, the input dataset using learned models to determine a clinical domain and relevant image annotations;
populating, by the computer, an annotation table using the relevant image annotations;
applying, by the computer, one or more domain-specific scriptable rules to populate a report template based on the annotation table; and
generating, by the computer, a natural language radiology report based on the report template.

2. The method according to claim 1, further comprising:
receiving, by the computer, an indication of a clinical study being performed on the input dataset,
wherein the natural language radiology report provides an explanation of a clinical finding relevant to the clinical study and one or more image features corresponding to the clinical finding.

3. The method according to 2, further comprising:
presenting the natural language radiology report in an interactive graphical user interface which allows a user to retrieve images depicting the one or more image features via activation of one or more links embedded in the natural language radiology report.

4. The method according to any of the preceding claims, wherein the natural language radiology report comprises one or more recommendations for modifying a scanner acquisition protocol to acquire one or more additional patient images.

5. The method of claim 4, further comprising:
receiving, by the computer, an indication of a clinical study being performed on the plurality of multidimensional patient images;
detecting, by the computer, that target anatomy relevant to the clinical study is partially or completely out of the field of view of all of the plurality of multidimensional patient images,
wherein the one or more recommendations for modifying the scanner acquisition protocol comprise a recommended modification to patient positioning during imaging.

6. The method according to any of the preceding claims, wherein a rule-out process is applied to the plurality of multidimensional patient images prior parsing the plurality of multidimensional patient images using the learned models, the rule-out process comprising:
receiving, by the computer, an indication of a clinical study being performed on the plurality of multidimensional patient images;
identifying, by the computer, a subset of the plurality of multidimensional patient images which are irrelevant to the clinical study; and
disregarding, by the computer, the subset of the plurality of multidimensional patient images from the input dataset or the input dataset as a whole.

7. The method according to any of the preceding claims, further comprising:
performing an offline preparation process comprising:
creating a clinical report template based on existing clinical reports and domain knowledge;
identifying one or more clinical report concepts and acceptable data ranges relevant to the clinical report concepts based on the existing clinical reports and the domain knowledge;
using the clinical report template, the one or more clinical report concepts, and the acceptable data ranges relevant to the clinical report concepts to create an annotation specification comprising one or more annotation tables and the one or more domain-specific scriptable rules;
using the annotation specification to create an annotation system;
applying the annotation system to one or more training images to yield one or more training image annotations; and
training one or more image parsing models based on the training images and the training image annotations.

8. The method according to claim 7, wherein the domain knowledge comprises one or more of clinical standards, clinical guidelines, or information provided in clinical consults.

9. The method according to claims 7 or, 8 further comprising:
using a basic report template provided in a Radiological Society of North America standardized format to create the clinical report template based on the existing clinical reports and domain knowledge.

10. A computer-implemented method for automatically generating a radiology report, the method comprising:
performing, by a computer, an offline training process comprising:
determining a plurality of possible image annotations associated with a clinical domain;
determining a plurality of scriptable rules for populating a domain-specific clinical report template with information relevant to the plurality of possible image annotations;
training one or more domain specific models to parse image information and output one or more of the plurality of possible image annotations; and performing, by the computer, an online report generation process comprising:
receiving an input dataset comprising a plurality of multidimensional patient images and patient information;
deriving one or more relevant image annotations associated with the input dataset based on the plurality of possible image annotations associated with the clinical domain and the one or more domain specific models;
populating the domain-specific clinical report template using the one or more relevant image annotations and the plurality of scriptable rules; and
identifying one or more clinically relevant findings based on the populated domain-specific clinical report template.

11. The method according to claim 10, wherein the online report generation process further comprises:
generating a natural language radiology report based on the one or more clinically relevant findings.

12. The method according to claim 11, wherein the online report generation process further comprises:
presenting the natural language radiology report in an interactive graphical user interface which allows a user to retrieve images depicting image features relevant to the clinically relevant findings via activation of one or more links embedded in the natural language radiology report.

13. The method according to any of the preceding claims 10 to 12, wherein the online report generation process further comprises:
identifying a change to an existing image acquisition protocol based on the one or more clinically relevant findings.

14. The method according to claim 13, wherein the online report generation process further comprises:
automatically implementing the change to the existing image acquisition protocol on an image scanner to acquire one or more new images.

15. The method according to claim 13 or 14, wherein the online report generation process further comprises:
displaying the change to the existing image acquisition protocol in a graphical user interface as a recommendation to a user.

16. The method of claim 15, wherein the online report generation process further comprises:
detecting, by the computer, that target anatomy relevant to the clinical domain is partially or completely out of the field of view of all of the plurality of multidimensional patient images,
wherein the change to the existing image acquisition protocol comprises a recommended modification to patient positioning during imaging.

17. The method according to any of the preceding claims 13 to 16, wherein the online report generation process further comprises:
identifying, by the computer, a subset of the plurality of multidimensional patient images which are irrelevant to the clinical domain based on the one or more clinically relevant findings; and
disregarding, by the computer, the subset of the plurality of multidimensional patient images from the input dataset or the input dataset as a whole.

18. A system for automatically generating a radiology report, the system comprising:
a medical information database comprising one or more diagnostic multidimensional (e.g. 2D/3D/4D) image data and non-image patient metadata;
one or more processors configured to:
communicate with the medical information database to retrieve a patient-specific input dataset;
parse the patient-specific input dataset using learned models to determine a clinical domain and relevant image annotations;
populate an annotation table using the relevant image annotations;
apply one or more domain-specific scriptable rules to populate a report template based on the annotation table; and
identify one or more clinically relevant findings based on the populated report template.

19. The system according to claim 18, wherein the one or more processors are further configured to:
generate a natural language radiology report based on the report template.

20. The system according to claim 19, wherein the one or more processors are further configured to:
present the natural language radiology report in an interactive graphical user interface which allows a user to retrieve images depicting image features relevant to the clinically relevant findings via activation of one or more links embedded in the natural language radiology report.
